# EUROPÄISCHE PATENTSCHRIFT

(11) **EP 2 540 244 B1**
(45) Veröffentlichungstag und Bekanntmachung des Hinweises auf die Patenterteilung: **16.08.2017**
(21) Anmeldenummer: 11172084.3
(22) Anmeldetag: 30.06.2011
(51) Int. Cl.: A61B 18/12

(54) **Vorrichtung zum optimierten Koagulieren von biologischem Gewebe**
Device for optimised coagulation of biological tissue
Dispositif de coagulation optimisée de tissu biologique

(43) Veröffentlichungstag der Anmeldung: 02.01.2013
(73) Patentinhaber: Erbe Elektromedizin GmbH, 72072 Tübingen (DE)
(72) Erfinder: Werner, Erich, 72827 Wannweil (DE)
(74) Vertreter: Rüger, Rudolf

(56) Entgegenhaltungen:
- EP-A1- 2 213 255
- DE-A1-102004 041 681
- US-A1- 2002 151 884
- US-A1- 2003 158 551

## Beschreibung

Die Erfindung betrifft ein System zum Koagulieren von biologischem Gewebe durch Einleiten von elektrischem Strom in dasselbe.

Die elektrische Gewebekoagulation hat sich in der chirurgischen Praxis als probates Mittel zur Beseitigung von unerwünschten Gewebevolumina entwickelt. Zur Koagulation wird hochfrequenter elektrischer Strom eingesetzt. Zur Festlegung desselben existieren verschiedene Verfahren.

Die EP 1 886 636 A1 zeigt ein solches Verfahren. Es beruht darauf, zunächst ein konstantes elektrisches Signal, z.B. eine konstante Spannung, an das Gewebe anzulegen, um seinen Anfangswiderstand zu ermitteln. Diese Phase kann z.B. 2 bis 3 Sekunden dauern und erfolgt vor der eigentlichen elektrochirurgischen Behandlung. Anstelle einer konstanten Spannung werden zur Messung des anfänglichen Gewebewiderstands auch andere konstante Größen genannt, wie bspw. konstante Leistung, konstanter Strom oder Energie. Nach Start der Behandlung werden der anfängliche Gewebewiderstand, der sich einstellende Impedanzrückgang, das Impedanzminimum und der erste Impedanzanstieg aufgezeichnet und analysiert. Aus diesen Größen können Rückschlüsse auf die Verhältnisse an den Elektroden und den Gewebetyp sowie das Feuchtigkeitsniveau desselben gezogen werden. Entsprechend werden Behandlungsparameter festgelegt, z.B. hinsichtlich der Behandlungsdauer oder der zu applizierenden Energie. Dazu wird eine Nachschlagtabelle herangezogen, die in einem Speicher abgelegt ist. Ein Mikrocontroller nutzt die aus der Nachschlagtabelle erhaltenen Behandlungswerte und legt diese der nachfolgenden Behandlung zugrunde.

Die EP 2 213 255 A1 offenbart ein mehrschrittiges Verfahren zur Einleitung von Energie in ein biologisches Gewebe. In einem ersten Schritt wird Energie in das Gewebe eingeleitet. Es wird ein interessierender Gewebezustand, z.B. der Gewebewiderstand über der Temperatur, überwacht und die Richtung der Änderung dieses Zustandes erfasst. Durch fortwährendes Nachregulieren des Energieeintrags wird versucht, diejenige Gewebetemperatur einzustellen, bei der sich der minimale Gewebewiderstand ergibt. Dazu sind zwei Regelschleifen implementiert, die für den steigenden und den fallenden Kennlinienteil gegensätzlich arbeiten.

DE102004041681 offenbart eine Vorrichtung zum Veröden von Gewebe unter Berücksichtigung der zeitlichen Änderung des Gewebewiderstandes.

Chirurgische Operationen sind häufig zeitkritisch. Es wird deshalb gewünscht, Koagulationsvorgänge bei hoher Qualität und Reproduzierbarkeit in möglichst kurzer Zeit abzuschließen.

Davon ausgehend ist es Aufgabe der Erfindung, ein verbessertes Koagulationsverfahren und eine verbesserte Koagulationseinrichtung anzugeben.

Diese Aufgabe wird mit der Einrichtung dem Verfahren nach Anspruch 1 gelöst.

Außerdem wird dic Aufgabe mit der Einrichtung nach An opruch 15 gclöot.

Dao Die erfindungsgemäße Vcrfahrcn Einrichtung beginnt ohne Vorschalten einer Messphase mit dem Anlegen einer koagulationswirksamen HF-Spannung an das biologische Gewebe zur Einleitung der Koagulation. Die bestimmte HF-Spannung kann im einfachsten Falle eine konstante Spannung sein. Es kann als bestimmte HF-Spannung auch ein bestimmter zeitlicher Spannungsverlauf vorgegeben werden, der bspw. mindestens einen Spannungsanstieg und/oder auch mindestens einen Spannungsabfall umfasst, beispielsweise kann die bestimmte HF-Spannung einer vorgegebenen Spannungskurve folgen.

Als anfänglich angelegte bestimmte Spannung kann, wie erwähnt, eine vorgegebene konstante HF-Spannung genutzt werden. Diese hat vorzugsweise einen Spitzenwert von höchstens 200V, was Funkenbildung und die Ausbildung eines Plasmas und damit schnelles Austrocknen des Gewebes vermeidet.

Während des Anlegens der bestimmten HF-Spannung wird die Tendenz des elektrischen Gewebewiderstands überwacht. Der Gewebewiderstand ist je nach Ausführungsform bei allen nachstehend beschriebenen Varianten der ohmsche Widerstand des Gewebes, der komplexe Scheinwiderstand des Gewebes, der Betrag des komplexen Scheinwiderstands, der Realteil der Gewebeimpedanz oder der Imaginärteil der Gewebeimpedanz.

Die Überwachung des Gewebewiderstands kann fortwährend, d.h. kontinuierlich oder auch in Zeitabständen, z.B. in ständiger Wiederholung erfolgen. Die Zeitabstände können nach Bedarf konstant oder unterschiedlich gewählt werden. Zum Beispiel können sie wenige Millisekunden betragen.

Wird eine Anstiegstendenz des Gewebewiderstands festgestellt, wird die HF-Spannung so reguliert, dass der elektrische Gewebewiderstand bestimmten Kriterien genügt. Diese bestimmten Kriterien können bspw. ein gewünschter zeitlicher Verlauf, das Einhalten eines konstanten Werts oder auch lediglich das Kriterium sein, dass der Gewebewiderstand einen bestimmten Maximalwert nicht überschreitet oder dass er in einem vorgegebenen Bereich liegt. Mit diesem Maßnahmen lässt sich in kurzer Zeit ein hoher definierter Energieeintrag erreichen, ohne das Gewebe vorzeitig auszutrocknen. Es wird eine schnelle Koagulation erreicht, die von hoher Qualität ist.

Durch die Festlegung eines bestimmten, großen Energieeintrags in das Gewebe kann sichergestellt werden, dass ein entsprechend großes Koagulationsvolumen erzielt wird, wobei auch ein Ankleben oder Austrocknen des Gewebes an der genutzten Elektrode des chirurgischen Instruments sicher ausgeschlossen werden kann. Die Beschränkung des Gewebewiderstands auf nicht zu hohe Werte, d.h. die Verhinderung des Anstiegs des Gewebewiderstands, ist ein sicherer Schutz gegen Austrocknen und Ankleben des Gewebes an der Elektrode. Somit schafft die Erfindung reproduzierbare Koagulationsergebnisse in hoher Qualität und kurzer Behandlungszeit.

Die Überwachung der Tendenz des elektrischen Gewebewiderstands kann durch fortwährende Widerstandsbestimmung und Vergleich mit einem Grenzwert, einem Vergleichswert, einem Toleranzband oder dergleichen erfolgen. Der Vergleichswert kann ein fest vorgegebener Wert oder auch ein zuvor gemessener Wert oder auch ein Durchschnittswert sein, der laufend, z.B. aus den letzten Messwerten, gebildet wird. Eine ansteigende Tendenz des Gewebewiderstands kann bspw. festgestellt werden, wenn der aktuelle Wert des Gewebewiderstands den Vergleichswert übersteigt. Anders als die Festlegung starrer Grenzen für den Gewebewiderstand wird auf diese Weise eine Anpassung an individuelle Gewebemerkmale ermöglicht.

Als der gewünschte Gewebewiderstand kann auch der im Widerstandsverlauf festgestellte minimale Wert des Gewebewiderstands oder ein im festen Verhältnis dazu festgelegter Wert genutzt werden. Beispielsweise kann ein fester prozentualer Aufschlag von z.B. 20% auf den gemessenen minimalen Gewebewiderstand gemacht werden und der so erhaltene Widerstandswert als Soll- oder Grenzwert für den Gewebewiderstand genutzt werden, der als Sollwert für die Einstellung des Gewebewiderstands genommen wird. Es ist auch möglich, anstelle eines festgelegten Sollwerts für den Gewebewiderstand einen festgelegten Zeitverlauf zu nutzen.

Es ist möglich, den Koagulationsprozess auf Basis der Regelung des Gewebewiderstands bis zu einem physiologischen Gleichgewicht an der Koagulationsgrenze fortzusetzen. So können sehr große Gewebevolumina koaguliert werden. Es ist aber auch möglich, die in das Gewebe eingeleitete Energie zu erfassen und auf einen maximalen Energiebetrag zu begrenzen. Weil bei dem erfindungsgemäßen Verfahren das koagulierte Volumen in sehr gute Näherung proportional zum Energieeintrag ist, kann dadurch das koagulierte Volumen beeinflusst werden. Zum Beispiel kann der Chirurg auf diese Weise das zu koagulierende Volumen als Eingabeparameter an einem medizinischen Gerät vorgeben und dann gewissermaßen blind arbeiten. Dies ist insbesondere dann vorteilhaft, wenn größere Volumina und größere Gewebetiefen zu koagulieren sind. Die erreichte Koagulationstiefe ist für den Chirurgen naturgemäß nur schwer erkennbar. Hier hilft ihm die Begrenzung der eingeleiteten Energie.

Nach Applikation der festgelegten Energiemenge kann die an der Elektrode anliegende Spannung auf einem minimalen, nicht zu einer Koagulation führenden Wert vermindert werden. Die Spannung kann eine HF-Spannung, eine NF-Spannung, eine Wechselspannung beliebiger Kurvenform oder eine Gleichspannung sein. Durch Überwachung des Stromflusses kann ein erneuter Gewebekontakt des Instruments erkannt und eine neue Behandlung eingeleitet werden.

Weitere Einzelheiten vorteilhafter Ausführungsformen der Erfindung sind Gegenstand von Unteransprüchen, der Zeichnung oder der Beschreibung. Es zeigen:
Figur 1 ein elektrochirurgisches Gerät und ein zugehöriges Instrument bei der Koagulation biologischen Gewebes, in schematisierter Darstellung,
Figur 2 das biologische Gewebe während der Koagulation, in symbolischer Darstellung,
Figur 3 mögliche zeitabhängige Verläufe des Gewebewiderstands als Diagramm,
Figur 4 den Verlauf des Gewebewiderstands und der angelegten HF-Spannung bei dem erfindungsgemäßen Verfahren,
Figur 5 eine Blockdarstellung eines Teils der Steuerung eines Geräts zur Umsetzung des erfindungsgemäßen Verfahrens und
Figur 6 ein Diagramm zur Veranschaulichung der Überwachung des Gewebewiderstands in zeitdiskretem Betrieb als Diagramm.

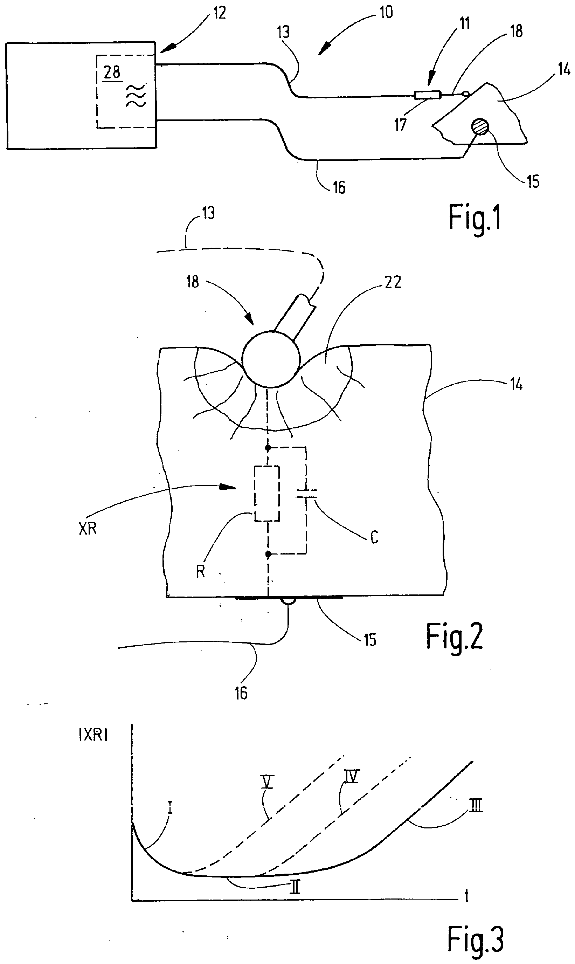

In Figur 1 ist eine Einrichtung 10 zur elektrochirurgischen Behandlung menschlicher oder tierischer Patienten veranschaulicht, zu der ein chirurgisches Instrument 11 und ein Gerät 12 zur Speisung desselben mit hochfrequentem Strom gehören. Beide sind durch eine Leitung 13 miteinander verbunden. Das Instrument 11 dient zur zonenweisen Koagulation von biologischem Gewebe 14, das über eine Neutralelektrode 15 und eine entsprechende Neutralleitung 16 an das Gerät 12 angeschlossen ist. Das biologische Gewebe 14 dient in Figur 1 zur Symbolisierung eines Teils eines Patienten.

Das Gerät 12 umfasst einen HF-Generator 28 zur Erzeugung einer HF-Spannung ausreichender Amplitude (bspw. bis zu mehreren 100 V) und eine Steuereinrichtung für den HF-Generator 28. Bedienelemente und Anzeigeelemente, die an dem Gerät 12 zur Steuerung und Kontrolle des Betriebs der Einrichtung 10 vorgesehen sein können, sind in Figur 1 nicht weiter veranschaulicht.

Das Instrument 11 umfasst einen elektrisch isolierenden Handgriff 17 und eine elektrisch leitfähige Elektrode 18 zur direkten Einwirkung auf das Gewebe 14. Figur 2 veranschaulicht das Gewebe 14 und die mit dem Gewebe 14 zusammenwirkende Elektrode 18, die hier lediglich zur beispielhaften Veranschaulichung als Kugelelektrode veranschaulicht ist. Es können jedoch auch andere geeignete Elektrodenformen, insbesondere Platten, Messer, Schlingen oder dergleichen Anwendung finden.

Zwischen der Elektrode 18 und der Neutralelektrode 15 bildet das biologische Gewebe 14 einen stromleitfähigen Weg, dessen Impedanz als Gewebewiderstand XR bezeichnet wird. Dieser Gewebewiderstand XR weist einen in der Regel dominierenden ohmschen Anteil in Form eines ohmschen Widerstands R auf. Außerdem kann der Gewebewiderstand XR reaktive Komponenten umfassen, die in Figur 2 durch eine Kapazität C und ein gestricheltes Kondensatorsymbol veranschaulicht sind. Zusätzlich kann dieser Gewebewiderstand XR beziehungsweise die Gewebeimpedanz auch induktive Anteile umfassen, die in Reihe mit dem ohmschen Widerstand R zu verstehen sind. Die nachfolgenden Ausführungen nehmen den Gewebewiderstand XR allgemein in Bezug. Sie beziehen sich sowohl auf Ausführungsformen, in denen unter dem Gewebewiderstand XR der komplexe, aus dem ohmschen Widerstand R und der Kapazität C gebildete Widerstand verstanden wird, als auch auf Ausführungsformen, bei denen unter dem Gewebewiderstand XR lediglich der ohmschen Widerstand R, die Kapazität C oder eine aus beiden gebildete Größe verstanden, gemessen, ausgewertet, gesteuert oder geregelt usw. wird.

Figur 3 veranschaulicht den Betrag des Gewebewiderstands XR in Abhängigkeit von der Zeit bei Einleitung von HF-Leistung in das biologische Gewebe 14. Wie die Kurve I zeigt, fällt der Gewebewiderstand zunächst relativ schnell ab und erreicht einen niedrigen Wert im Bereich der Kurve II. Durch fortgesetzte Energiezufuhr denaturiert das Gewebe und trocknet aus, wodurch es bei der Kurve III zum Anstieg des Gewebewiderstands kommt. Wie gestrichelte Kurven IV und V veranschaulichen, kann diese Widerstandszunahme auch zu früheren Zeitpunkten erfolgen, wenn z.B. infolge einer höheren angelegten HF-Spannung mehr Leistung in das Gewebe 14 eingeleitet wird.

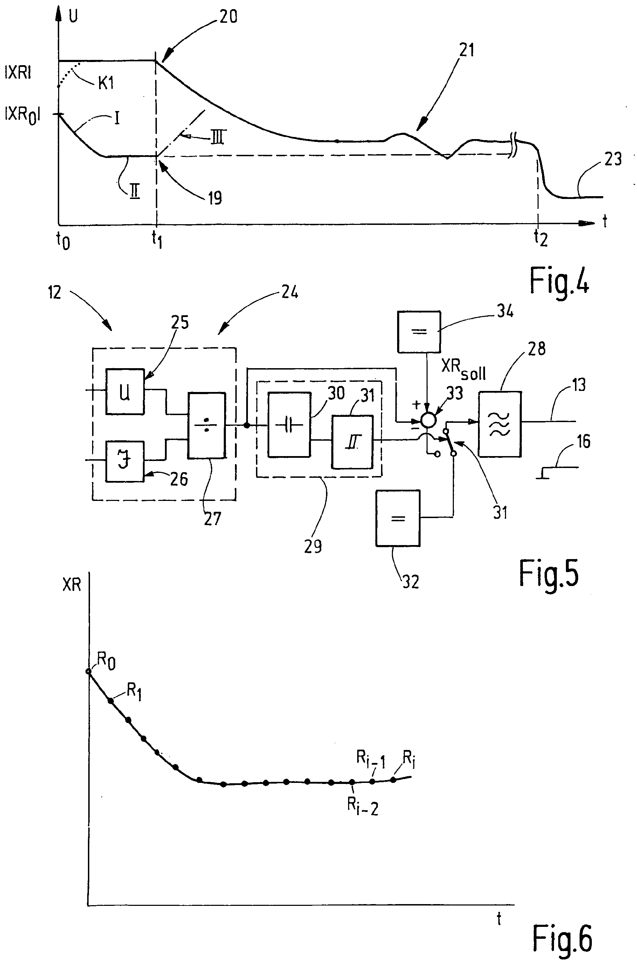

Figur 4 veranschaulicht den zeitlichen Verlauf des Betrags des Gewebewiderstands XR und den zugehörigen Verlauf des Spitzenwerts der HF-Spannung an der Elektrode 18 gegenüber der Neutralelektrode 15 bei Anwendung des erfindungsgemäßen Verfahrens:
Der Koagulationsprozess einer Zone beziehungsweise eines Volumens 22 (Figur 2) des biologischen Gewebes 14 beginnt zum Zeitpunkt t0. Zu diesem Zeitpunkt berührt die Elektrode 18 das Gewebe 14 und es liegt eine bestimmte HF-Spannung an der Elektrode 18 und somit an dem Gewebewiderstand XR an. Diese HF-Spannung ist im vorliegenden Beispiel eine sinusförmige Spannung mit einem Spitzenwert unterhalb von 200 V. Es können jedoch auch andere Spannungskurvenformen Anwendung finden. Z.B. kann die Sinusspannung gepulst sein, was zu einem Crestfaktor (Scheitelfaktor) von größer als Wurzel aus zwei führt. Abgesehen von dem dargestellten konstanten Wert kann die HF-Spannung auch auf andere Weise vorbestimmt sein und z.B. nach dem Einschaltzeitpunkt t0 auf einen konstanten Wert hin ansteigen, wie es durch eine gepunktete Kurve K1 veranschaulicht ist. Auch andere Spannungsverläufe sind anwendbar.

Während des Anliegens der konstant oder zeitvariabel vorgegebenen HF-Spannung U wird der Gewebewiderstand XR überwacht. Dabei wird festgestellt, dass der Gewebewiderstand XR zunächst mit der Kurve I abfällt und dann einen niedrigen Wert entsprechende Kurve II erreicht. Durch den hohen Energieeintrag aufgrund der hohen Leistung der eingeleiteten HF-Spannung hat der Gewebewiderstand XR alsbald die Tendenz wieder anzusteigen, was in Figur 4 durch die gestrichelte Kurve III veranschaulicht wird. Der Zeitpunkt, bei dem dies festgestellt wird, ist der Zeitpunkt t1.

Die Steuerung des Geräts 12 stellt zu dem Zeitpunkt t1 fest, dass die Kurve II des Gewebewiderstands XR bei der Stelle 19 in die Kurve III übergehen will. Der HF-Generator des Geräts 12 wird nun vom Betrieb mit vorgegebener Spannung auf einen Betrieb umgestellt, in dem die HF-Spannung U so geführt wird, dass der Gewebewiderstand XR nicht oder nicht wesentlich ansteigt, sondern vielmehr zumindest nahezu konstant bleibt. Die Umschaltung der Betriebsart erfolgt zu dem Zeitpunkt t1, was an dem Verlauf der Spannung an der Stelle 20 erkennbar ist. Die Leistungsabgabe des Geräts 12 wird dadurch so reguliert, dass der Gewebewiderstand XR nicht über seinen Ausgangswert |XR₀| ansteigt. Damit wird ein vorzeitiges Trocknen und Ankleben des Gewebes an der Elektrode 18 verhindert.

Zur Regelung des Gewebewiderstands XR sind, wie schon angedeutet mehrere Varianten möglich. Es kann der niedrigste gemessene Gewebewiderstand als Sollwert festgelegt werden. Die Regelung des Geräts 12 stellt dann diesen Gewebewiderstand ein. Es kann auch der niedrigste gemessene Gewebewiderstand, multipliziert mit einem gegebenen oder wählbaren Faktor als Sollwert festgelegt werden. Auch kann der niedrigste gemessene Gewebewiderstand, vergrößert um einen gegebenen oder wählbaren Summanden als Sollwert festgelegt werden. Alternativ kann der initiale Gewebewiderstand XR₀, multipliziert mit einem gegebenen oder wählbaren Faktor (der vorzugsweise kleiner als 1 ist) als Sollwert festgelegt werden. Weiter alternativ kann der initiale Gewebewiderstand XR₀, vermindert um einen gegebenen oder wählbaren Subtrahenden als Sollwert festgelegt werden. Es kann als Sollwert auch ein aus mehreren gemessenen Gewebewiderstandswerten errechneter Wert oder auch ein willkürlich festgelegter Wert dienen.

Im weiteren Verlauf kann sich die Spannung auch entsprechend der Arbeit des Operateurs im Rahmen des Regelprozesses ändern, wie in Figur 4 symbolisch an der Stelle 21 des Spannungsverlaufs angedeutet ist. Ein Regler hält jedoch den Gewebewiderstand XR möglichst konstant, jedenfalls aber unterhalb eines Grenzwertes. Als dieser Grenzwert kann der Sollwert dienen, der nach einem der obigen Verfahren bestimmt worden ist.

Es ist möglich, den Prozess bis zu einem physiologischen/thermischen Gleichgewicht fortzusetzen, bis der Chirurg das gewünschte max. mögliche Gewebevolumen koaguliert hat. Auf diese Weise können willkürlich gewählte auch beliebig große Gewebevolumina koaguliert werden. Es ist jedoch auch möglich, das Koagulationsvolumen zu begrenzen. Figur 2 veranschaulicht in dem Gewebe 14 das Volumen 22, das koaguliert werden soll. Die Beschränkung dieses Volumens 22 kann durch eine Steuerung und Begrenzung der von dem Gerät 12 abgegebenen Leistung erfolgen. Dazu wird bspw. die während der Phase zwischen den Zeitpunkten t0 und t1 sowie zusätzlich die danach abgegebene HF-Leistung aufsummiert (integriert). Erreicht der Wert der so ermittelten abgegebenen Energie einen Grenzwert, kann das Gerät 12 zu einem Zeitpunkt t2 die Abgabe der HF-Spannung unterbrechen, beenden oder bspw., wie in Figur 4 dargestellt, auch auf einen niedrigen physiologisch unwirksamen Wert 23 reduzieren. Damit ist der Koagulationsprozess beendet. Jedoch ist eine geringe an der Elektrode 18 vorhanden Gleich- oder Wechselspannung niederer oder hoher Frequenz vorhanden, um erfassen zu können, ob und wann die Elektrode 18 wiederum biologisches Gewebe 14 berührt. Solche Signale können zum Wiedereinschalten des Generators des Geräts 12 genutzt werden, bspw. um einen weiteren Koagulationsprozess nach vorstehendem Vorbild durchzuführen.

In Figur 5 wird ein Auszug aus einer beispielhaften Steuerung des Geräts 12 zur Durchführung wenigstens einer Variante des vorbeschriebenen Verfahrens veranschaulicht:
Die Steuerung enthält ein Modul 24 zur Erfassung des Gewebewiderstands XR. Dieses Modul 24 kann prinzipiell auf jede geeignete Weise ausgebildet sein. Das Modul 24 kann einen ersten Block 25 zur Erfassung der an der Elektrode 18 anliegenden HF-Spannung und einen Block 26 zur Erfassung des zu der Elektrode 18 fließenden Stroms umfassen. Die Module 25, 26 sind z.B. entsprechend an die zu dem Instrument 11 führenden Leitung 13 angeschlossen. Alternativ kann das Modul 26 auch an die Leitung 16 angeschlossen sein. An ihren Ausgängen liefern die Module 25, 26 entweder einen den Betrag oder den Effektivwert oder den aktuellen Zeitwert, der Spannung bzw. des Stroms kennzeichnendes Signal. Einweiterer Block 27 bildet den Quotienten aus den von den Blöcken 25, 26 gelieferten Signalen und liefert an seinem Ausgang somit je nach Ausbildung der Blöcke 25, 26 ein den komplexen Gewebewiderstand XR oder den ohmschen Anteil R des Gewebewiderstands kennzeichnendes Signal.

Zu dem Gerät 12 gehört außerdem der HF-Generator 28, der die Leitung 13 speist. Außerdem weist der HF-Generator 28 einen Steuereingang auf. Dort empfangene Steuersignale legen die Größe der an dem Ausgang des HF-Generators 28 anstehenden, an die Leitung 13 gelieferten Spannung fest.

An das Modul 24 ist ein Tendenzerkennungsmodul 29 angeschlossen. Dieses umfasst z.B. einen Block 30 zum Differenzieren des Ausgangssignals des Moduls 24. Der Block 30 liefert ein positives Ausgangssignal, wenn der Gewebewiderstand XR ansteigt und ein negatives (oder kein) Ausgangssignal, wenn der Gewebewiderstand XR absinkt. Ein Komparator 31 formt dieses Signal zu einem Schaltsignal um. Das Schaltsignal dient zum Umschalten des Betriebs des HF-Generators 28 mittels eines Schalters 31. Dieser verbindet den Steuereingang des HF-Generators 28 entweder mit einem von einem Block 32 gelieferten Spannungssollsignal oder mit dem Ausgangssignal eines Subtrahierers 33. Dieser subtrahiert das von dem Modul 24 gelieferte Widerstandssignal von dem von einem Block 34 gelieferten Signal, das den Sollwert XRsoll des Gewebewiderstands kennzeichnet. Die Abweichung zwischen dem realen Gewebewiderstand XR und dem Sollwert XRsoll, den der Block 34 vorgibt, wird somit zu einer Führungsgröße für die den HF-Generator 28 enthaltende Regelschleife.

Im einfachsten Fall ist der von dem Block 34 gelieferte Sollwert XRsoll ein fest vorgegebener Wert, der vorzugsweise unter dem initialen Gewebewiderstand XR₀ liegt. Der Wert XRsoll kann jedoch von dem Block 34 alternativ auch bestimmt und z.B. rechnerisch festgelegt werden. Dazu sind verschiedene Verfahren nutzbar. Zum Beispiel kann der Sollwert XRsoll gleich dem kleinsten zuvor gemessenen Gewebewiderstand XR sein. Alternativ kann der Wert XRsoll um einen Fixbetrag oder um einen Faktor höher sein als der kleinste zuvor gemessene Gewebewiderstand XR. Alternativ kann der Sollwert aus zuvor gemessenen Werten des Gewebewiderstands XR ermittelt werden, indem z.B. mehrere vorige Werte des Gewebewiderstands miteinander verrechnet werden. Zum Beispiel kann aus ihnen ein Durchschnitt gebildet werden, wobei dieser Durchschnittswert wiederum kleiner als der initiale Gewebewiderstand XR₀ ist. Auch kann der Block 34 einen gewünschten Zeitverlauf für XR vorgeben, bspw. in Gestalt eines zeitkonstanten Werts oder auch in Gestalt eines zeitlich variierenden Werts. Auch kann XRsoll nach einem der vorstehenden Verfahren anhand des initialen Gewebewiderstands XR₀ festgelegt werden.

Die vorstehend beschriebenen Module, Blöcke, Schalter, Subtrahierer und dergleichen können sowohl hardwaretechnisch als auch softwaretechnisch durch Programme oder Programmteile bspw. eines auf einem Mikrorechner laufenden Programms realisiert werden. Damit kann die Erfassung des Gewebewiderstands in dem Modul 24, wie vorstehend beschrieben, kontinuierlich oder auch fortlaufend wiederkehrend im Sinne einer Abtastung durchgeführt werden, wie es Figur 6 veranschaulicht. Der Gewebewiderstand XR nimmt dort im Verlaufe der Zeit verschiedene Werte ein, die jeweils durch Punkte gekennzeichnet sind. Zum Beispiel kann das Modul 24 so beschaffen sein, dass es den aktuellen Widerstandswert Rᵢ jeweils mit mindestens einem oder auch mehreren vorhergehenden Werten Rᵢ₋₁, Rᵢ₋₂, usw. vergleicht. Auch kann aus den vorhergehenden Werten Rᵢ₋₁, R₁₋₂, usw., ein laufender Durchschnittswert gebildet werden. Sollte Rᵢ größer sein als der laufende Durchschnittswert, ist eine ansteigende Tendenz erkennbar und es wird wiederum der Schalter 31 von Betrieb mit konstanter oder vorgegebener Spannung (Block 32) auf Betrieb mit konstantem Gewebewiderstand (Block 34) umgeschaltet.

Es wird nochmals darauf hingewiesen, dass im Rahmen der vorigen Beschreibung sowohl Geräte und Verfahren in betracht gezogen werden, bei denen zwischen den Zeitpunkten t0 und t1 fest vorgegebene konstante HF-Spannungen wie auch vorgegebene HF-Spannungsverläufe genutzt werden. Weiter werden für die vorigen Ausführungsformen sowohl Geräte und Verfahren in Betracht gezogen, bei denen nach dem Zeitpunkt t1 der komplexe Scheinwiderstand XR, der Betrag des Scheinwiderstands XR, der ohmsche Anteil R des Gewebewiderstands R oder ein sonstiges charakteristisches Kennzeichen des Gewebes 14 konstant gehalten wird. Weiter wird darauf hingewiesen, dass bei allen vorbeschriebenen Ausführungsformen eine Energiebegrenzung vorgesehen werden kann, bei der die insgesamt in das Gewebe 14 eingeleitete Energie erfasst und begrenzt wird, um das koagulierte Volumen 22 festzulegen. Alternativ kann auch lediglich der Energiebetrag erfasst und begrenzt werden, der ab dem Zeitpunkt t1 in das Gewebe eingeleitet worden ist.

Bei dem erfindungsgemäßen Verfahren wird bei einer vorgegebenen, vorzugsweisen konstanten maximalen Koagulationsspannung (<200 Vp) initial der maximale HF-Strom in das Gewebe 14 eingeprägt. In diesem initialen Zustand verhält sich das Gewebe gemäß dem ohmschen Gesetz und kann die maximale Energie pro Zeit aufnehmen. Nachdem das Gewebe aus einem initialen Zustand in einen Zustand gewechselt hat, in dem die Gewebeimpedanz oder der Gewebewiderstand XR spannungsabhängig ist, werden die HF-Parameter so gewählt, dass sich für den neuen Gewebezustand der maximal mögliche Energieeintrag pro Zeit einstellt. Dazu wird der Gewebewiderstand vorzugsweise unter dem Wert der Anfangsimpedanz gehalten. Ein Regler begrenzt den Gewebewiderstand entsprechend auf einen solchen Wert, der kleiner ist als die Anfangsimpedanz bzw. auf einen Wert der kleiner ist als die sich einstellende geringste Gewebeimpedanz. Erfindungsgemäß ist ein automatischer Aktivierungsabbruch vorgesehen , der erfolgt, wenn ein vorgegebener Energieeintrag erreicht ist. Dieser kann z.B. 10 Joule bis 500 Joule betragen.

### Bezugszeichenliste:

- 10: Einrichtung
- 11: Instrument
- 12: Gerät
- 13: Leitung
- 14: biologisches Gewebe
- 15: Neutralelektrode
- 16: Neutralleitung
- 17: Handgriff
- 18: Elektrode
- XR: komplexer Gewebewiderstand
- |XR|: Betrag des Gewebewiderstands
- R: ohmscher Widerstand
- C: Kapazität
- I - V: Kurven
- t0: Beginn des Koagulationsvorgangs
- K1: Kurve
- t1: Beginn der Gewebewiderstandsreglung
- t2: Ende des Koagulationsprozesses
- 19: Stelle der Kennlinie des Gewebewiderstands XR
- 20: Übergangsstelle des HF-Spannungsverlaufs
- |XR₀|: Betrag des Ausgangswerts des Gewebewiderstands
- 21: Stelle des Spannungsverlaufs
- 22: Volumen
- 23: niedriger Wert der HF-Spannung
- 24: Modul zur Erfassung des Gewebewiderstands XR
- 25: Block zur Spannungserfassung
- 26: Block zur Stromerfassung
- 27: Block
- 28: HF-Generator
- 29: Modul zur Erfassung der Tendenz von XR
- 30: Block zum Differenzieren von XR
- 31: Block zur Erzeugung eines Schaltsignals
- 32: Block zur Lieferung eines Spannungs-Soll-Signals
- 33: Subtrahierer
- 34: Block zur Lieferung eines Widerstands-Soll-Signals

## Patentansprüche

1. Einrichtung bestehend aus einem elektrochirurgischen Instrument (11) und einem Gerät (12) zu Speisung desselben mit HF-Spannung zum Koagulieren von biologischem Gewebe (14) durch Einleiten von elektrischem Strom in dasselbe mittels eines chirurgischen Instruments (11), wobei die Einrichtung folgende Schritte ausführt:
Anlegen einer vorgegebenen bestimmten konstanten HF-Spannung an das Gewebe (14) zur Einleitung der Koagulation, und dabei:
Überwachen der Tendenz des elektrischen Gewebewiderstands (XR), sowie,
nachdem das Gewebe aus einem initialen Zustand in einen Zustand gewechselt hat, in dem die Gewebeimpedanz oder der Gewebewiderstand XR spannungsabhängig ist, Regulierung der HF-Spannung zur Begrenzung oder Einstellung des elektrischen Gewebewiderstands (XR) auf einen gewünschten Wert (XRsoll), sofern eine steigende Tendenz des Gewebewiderstands (XR) festgestellt worden ist, **gekennzeichnet dadurch, dass** als der gewünschte Wert des Gewebewiderstands (XR) ein fest vorgegebener Wert (XRsoll), ein zuvor gemessener Wert des Gewebewiderstands (XR) oder ein aus zuvor gemessenen Werten ermittelter Sollwert genutzt wird,
wobei die in das Gewebe (14) eingeleitete Energie erfasst und die HF-Spannung auf einen minimalen Wert reduziert wird, wenn ein festgelegter Energiebetrag erreicht ist, um den Energieeintrag zu begrenzen.

2. Einrichtung nach Anspruch 1, **dadurch gekennzeichnet, dass** die bestimmte HF-Spannung einen Spitzenwert von höchstens 200 V aufweist.

3. Einrichtung nach Anspruch 1, **dadurch gekennzeichnet, dass** die Überwachung der Tendenz des elektrischen Gewebewiderstands (XR) erfolgt, indem fortwährend der aktuelle Wert des Gewebewiderstands (XR) bestimmt und mit einem Vergleichswert verglichen wird.

4. Einrichtung nach Anspruch 3, **dadurch gekennzeichnet, dass** der Vergleichswert ein zuvor gemessener Wert oder ein Durchschnittwert ist, der aus mehreren zuvor gemessenen Werten (Rᵢ₋₁, Rᵢ₋₂ ...) bestimmt wird.

5. Einrichtung nach Anspruch 3 oder 4, **dadurch gekennzeichnet, dass** die steigende Tendenz des Gewebewiderstands (XR) festgestellt wird, wenn der aktuelle Wert den Vergleichswert übersteigt.

6. Einrichtung nach Anspruch 1, **dadurch gekennzeichnet, dass** als der gewünschte Wert des Gewebewiderstands (XR) ein minimaler Wert des Gewebewiderstands (XR) genutzt wird.

7. Einrichtung nach Anspruch 1, **dadurch gekennzeichnet, dass** als der gewünschte Wert des Gewebewiderstands (XR) ein festgelegter Soll-Wert oder ein festgelegter Zeitverlauf des Werts genutzt wird.

8. Einrichtung nach Anspruch 1, **dadurch gekennzeichnet, dass** die Erfassung der Energie beginnt, sobald die Regulierung der HF-Spannung begonnen hat.

9. Einrichtung nach Anspruch 1, **dadurch gekennzeichnet, dass** der Energiebetrag festlegbar ist.

10. Einrichtung nach Anspruch 1, **dadurch gekennzeichnet, dass** der minimale Wert der HF-Spannung ein niedriger, nicht zu einer Koagulation führender Wert ist.

11. Einrichtung nach Anspruch 10, **dadurch gekennzeichnet, dass** bei minimaler HF-Spannung der Stromfluss überwacht wird, um einen Gewebekontakt des Instruments (11) zu erkennen.

## Claims

1. Apparatus comprising an electrico-surgical instrument (11) and a device (12) for supplying the same with HF voltage for coagulating biological tissue (14) by introducing electrical current therein by means of a surgical instrument (11), wherein the apparatus executes the following steps:
- application of a predefined specific constant HF voltage to the tissue (14) for initiating coagulation, and at the same time:
- monitoring the tendency of the electrical tissue resistance (XR), and
- after the tissue has changed from the initial state into a state in which the tissue impedance or tissue resistance XR is voltage-dependent, regulating the HF voltage to limit or set the electrical tissue resistance (XR) to a desired value (XRsoll) insofar as a rising tendency of tissue resistance (XR) has been established,
**characterised in that** the desired value of tissue resistance (XR) is a fixedly predefined value (XRsoll), a previously measured value of tissue resistance (XR) or a setpoint derived from the previously measured values,
wherein the energy introduced into the tissue (14) is detected and the HF voltage reduced to a minimum value when an established energy amount has been reached, in order to limit the energy input.

2. Apparatus according to claim 1, **characterised in that** the specific HF voltage has a peak value of maximum 200 V.

3. Apparatus according to claim 1, **characterised in that** the tendency of the electrical tissue resistance (XR) is monitored **in that** the present value of the tissue resistance (XR) is determined continuously and compared with a comparison value.

4. Apparatus according to claim 3, **characterised in that** the comparison value is a previously measured value or an average value which is determined from several previously measured values (Rᵢ₋₁, Rᵢ₋₂,...).

5. Apparatus according to claim 3 or 4, **characterised in that** the rising tendency of the tissue resistance (XR) is established when the present value exceeds the comparison value.

6. Apparatus according to claim 1, **characterised in that** a minimum value of tissue resistance (XR) is used as a desired value of tissue resistance (XR).

7. Apparatus according to claim 1, **characterised in that** an established setpoint or an established time development of the value is used as a desired value of tissue resistance (XR).

8. Apparatus according to claim 1, **characterised in that** detection of the energy begins as soon as regulation of the HF voltage has begun.

9. Apparatus according to claim 1, **characterised in that** the energy amount can be determined.

10. Apparatus according to claim 1, **characterised in that** the minimum value of the HF voltage is a low value not leading to coagulation.

11. Apparatus according to claim 10, **characterised in that** at the minimum HF voltage, the current flow is monitored in order to detect tissue contact of the instrument (11).

## Revendications

1. Dispositif constitué d'un instrument électro-chirurgical (11) et d'un appareil (12) permettant d'alimenter celui-ci avec une tension haute fréquence, et servant à faire coaguler un tissu biologique (14) en y faisant passer un courant électrique au moyen de l'instrument chirurgical (11), lequel dispositif exécute les opérations suivantes :
- appliquer une tension HF spécifiée constante prédéterminée au tissu (14), pour en amorcer la coagulation,
- surveiller comment évolue la résistance électrique (XR) du tissu,
- et après que le tissu est passé d'un état initial à un état dans lequel l'impédance ou la résistance XR du tissu dépend de la tension, réguler la tension HF de manière à limiter ou ajuster la résistance électrique XR du tissu à une valeur voulue (XR de consigne), si l'on a constaté que la résistance XR du tissu a tendance à augmenter,
**caractérisé en ce qu'**est utilisée, en tant que valeur voulue de la résistance XR du tissu, une valeur préalablement bien établie (XR de consigne), une valeur de la résistance XR du tissu mesurée auparavant, ou une valeur de consigne déterminée à partir de valeurs mesurées auparavant,
étant entendu que l'énergie apportée dans le tissu (14) est enregistrée et que l'on abaisse la tension HF à une valeur minimale quand est atteinte une quantité totale d'énergie déterminée, afin de limiter l'apport d'énergie.

2. Dispositif conforme à la revendication 1, **caractérisé en ce que** la tension HF spécifiée présente une valeur de crête d'au plus 200 V.

3. Dispositif conforme à la revendication 1, **caractérisé en ce que** surveiller comment évolue la résistance électrique (XR) du tissu consiste à déterminer en continu la valeur effective de la résistance XR du tissu et à la comparer avec une valeur de référence.

4. Dispositif conforme à la revendication 3, **caractérisé en ce que** la valeur de référence est une valeur mesurée auparavant, ou une valeur moyenne qui est déterminée à partir de plusieurs valeurs (Rᵢ₋₁, Rᵢ₋₂, ...) mesurées auparavant.

5. Dispositif conforme à la revendication 3 ou 4, **caractérisé en ce que** la tendance de la résistance XR du tissu à augmenter est constatée quand la valeur effective dépasse la valeur de référence.

6. Dispositif conforme à la revendication 1, **caractérisé en ce qu'**est utilisée, en tant que valeur voulue de la résistance XR du tissu, une valeur minimale de la résistance XR du tissu.

7. Dispositif conforme à la revendication 1, **caractérisé en ce qu'**est utilisée, en tant que valeur voulue de la résistance XR du tissu, une valeur de consigne bien établie ou une évolution bien établie de la valeur dans le temps.

8. Dispositif conforme à la revendication 1, **caractérisé en ce que** l'enregistrement de l'énergie commence dès que la régulation de la tension HF a commencé.

9. Dispositif conforme à la revendication 1, **caractérisé en ce que** la quantité totale d'énergie peut être déterminée..

10. Dispositif conforme à la revendication 1, **caractérisé en ce que** la valeur minimale de la tension HF est une valeur relativement basse qui n'entraîne pas de coagulation.

11. Dispositif conforme à la revendication 10, **caractérisé en ce que**, à la tension HF minimale, le courant est surveillé pour que soit vérifié que l'instrument (11) est au contact du tissu.
